# EUROPEAN PATENT SPECIFICATION

(11) **EP 3 250 117 B1**
(45) Date of publication and mention of the grant of the patent: **18.09.2019**
(21) Application number: 16742892.9
(22) Date of filing: 28.01.2016
(51) Int. Cl.: A61B 5/01, A61B 5/024, A61B 5/08

(54) **ORALLY INSERTED PROBE AND METHOD FOR MEASURING VITAL SIGNS**
ORAL EINGEFÜHRTE SONDE UND VERFAHREN ZUM MESSEN DER VITALZEICHEN
SONDE À INSERTION BUCCALE ET PROCÉDÉ DE MESURE DE PARAMÈTRES VITAUX

(30) Priority: 28.01.2015 US 201562108541 P
(43) Date of publication of application: 06.12.2017
(73) Proprietor: Respidx Ltd., 6962426 Tel Aviv (IL)
(72) Inventor: ALDAR, Yossi, 6962426 Tel-Aviv (IL)
(74) Representative: Ferrari, Barbara
(86) International application number: PCT/IL2016/050090
(87) International publication number: WO 2016/120875

(56) References cited:
- WO-A1-01/58349
- US-A1- 2005 096 558
- US-A1- 2005 096 558
- US-A1- 2012 165 623
- US-A1- 2013 253 286

## Description

### FIELD OF THE INVENTION

The present invention generally pertains to an orally inserted probe and a method for measuring vital signs.

### BACKGROUND TO THE INVENTION

Measuring and monitoring vital signs, such as respiratory rate, heart rate, oxygen saturation, and body temperature, is a key factor in diagnosis and management of numerous infectious diseases.

Monitoring respiratory rate is especially important when treating respiratory conditions such as pneumonia, in which a reduction in lung capacity causes a mammalian subject to breathe more rapidly in order to increase body oxygenation. In many cases, detecting a rapid respiratory rate can be the sole indicator of this condition.

Measuring respiratory rate is often complicated by the mammalian subject's breathing through their nose or their mouth, or alternating between the two. A reading of respiratory rate is typically performed by medical personnel such as a trained doctor or nurse, using a stethoscope and a clock. This method requires a skill level generally not present in the home environment or in the ambulatory environment.

It is also well known that many medical conditions, such as pneumonia, are characterized by low oxygen saturation. Furthermore, many medical conditions are accompanied by too rapid a heart rate (tachycardia) or too slow a heart rate (bradycardia). Thus, regular pulse oximetry readings of oxygen saturation and heart rate also provide a useful tool for screening for disease.

It is also well known that many medical conditions are associated with increased body temperature. This is particularly the case with infectious diseases such as pneumonia. Thus regular measurements of body temperature are also a useful screening tool for these diseases.

U.S. Patent Application Publication US2005/0096558 A1 discloses a temperature and respiration acquisition apparatus and method.

There is therefore a long-felt but unmet need for a means for unskilled persons to measure vital signs such as respiratory rate, temperature, oxygen saturation and heart rate in situations where medical personnel are not present; for example, in home care or ambulatory care. There is also such a need in developing countries, where medical personnel typically undergo far less training than their developed-nation counterparts.

### SUMMARY OF THE INVENTION

It is therefore the objective of the present invention to enable simple determination of vital signs by an untrained caregiver.

It is a further objective of the invention to provide a small, easy-to-use device to achieve said objective.

It is a further objective of the invention to ensure that the device is ergonomic, anatomically conformed, and safe to use, even for an infant mammalian subject.

It is a still further objective of the invention to measure at least two vital signs simultaneously, for example temperature together with the mammalian subject's respiratory rate, or temperature together with the vital signs measurable via pulse oximetry (i.e. heart-rate and oxygen saturation).

It is a still further objective of the invention to enable data storage of the measured vital signs data.

It is a still further objective of the invention to communicate the measured vital signs data to one or more devices located nearby and/or remotely.

The current invention relates to a probe [100] for measuring vital signs of a mammalian as defined in claims 1-8. In addition, the invention relates to a method for measuring vital signs as defined in claims 9-15.

### BRIEF DESCRIPTION OF THE FIGURES

Figure **1** is an external view of the probe according to some embodiments of the invention.
Figure **2** is a mechanical sketch of the probe showing features inside the probe, according to some embodiments of the invention.
Figure **3A** is a frontal view of the probe (i.e. from a side of the probe facing the mammalian subject), according to some embodiments of the invention. Figure **3B** is a side view of the probe while in use by a mammalian subject.
Figure **4A** is a frontal view of the probe, according to some alternative embodiments of the invention. Figure **4B** is a side view of the probe while in use by a mammalian subject.
Figure **5** shows a method for measuring vital signs of a mammalian subject, according to some embodiments of the invention.
Figure **6** shows a method for measuring respiratory rate of a mammalian subject, according to some embodiments of the invention.
Figure **7** shows a waveform of respiratory sensor reading measurements, collected and analyzed during measuring respiratory rate of a mammalian subject, according to some embodiments of the invention.
Figure **8** shows a method of sharing vital-sign data with others and utilizing the vital-sign data, according to some embodiments of the invention.
Figure **9A** is a block diagram of sensors and electronic sections of a probe according to some embodiments of the invention; Figure **9B** is an exploded view showing a PCB of the probe.

### DETAILED DESCRIPTION

### Overview

There is provided by the present invention a probe and method for measuring one or more vital signs of a mammalian subject. The mammalian subject can be a human being, especially including an infant or young child.

Means and methods for measuring temperature at the mouth are known, but the measurement of additional vital signs of a mammalian subject using a probe with a mouthpiece resembling a familiar oral thermometer is a novel concept. Key elements appearing in a probe according to one or more embodiments of the invention are as follows:
1. Incorporation of extra-oral sensors into the probe, deployed in the area of the mammalian subject's lip and near the nose when the mouthpiece of the apparatus is inserted (like a thermometer) into the mammalian subject's mouth. The mouthpiece is inserted and held in place in the mouth, and a stopper or protrusion helps to maintain correct positioning of the extra-oral sensors. This design approach enables the probe to measure additional vital signs, while, like a traditional oral thermometer, taking the mammalian subject's temperature using a temperature sensor in the mouthpiece.
2. One type of extra-oral sensors of the probe is placed close to the nostrils and detects a physical property changing with inspiration/expiration cycle of inhaled/exhaled air, enabling the mammalian subject's respiratory rate to be measured.
3. A reflective pulse oximetry sensor to measure heart rate and oxygen saturation is available, and the use of such a sensor on the forehead is well known; surprisingly it is herein shown that it is also possible to use such a sensor at the lip. It is a feature of the present invention to provide novel embodiments which deploy a reflective pulse oximetry sensor in the probe to take a pulse-oximetry reading.

The probe of the present invention is able to measure up to four vital signs simultaneously: body temperature, respiratory rate, heart rate and blood oxygenation, thereby enabling a new form of "thermometer" which also provides multiple vital signs measurements. The respiratory sensors and pulse oximetry sensor can be held in place by a part of the probe that, with the mouthpiece, further enhances positional stability of the extra-oral sensors, including but not limited to: 1) mounted on a protrusion extending from the apparatus, attached to the probe and seated against the mammalian subject's outer lip when the mouthpiece is inserted; 2) incorporated into a skirt of the mouthpiece, seated against the mammalian subject's outer lip and substantially covering the opening of the mammalian subject's mouth; 3) mounted on a sliding section which is pushed towards the lip once the mouthpiece is inserted into the mammalian subject's mouth; or 4) incorporated into a flexible arm which presses the probe against the lip.

In an exemplary embodiment of the invention, a probe for measuring vital signs of a mammalian subject includes at least one vital-sign sensor. A vital-sign sensor can be one or more of respiratory sensors for measuring respiratory rate and a pulse oximetry sensor for measuring heart rate and oxygen saturation. The vital-sign sensors are in mechanical connection with a mouthpiece of the probe. The mouthpiece can resemble a familiar, easy-to-use digital oral thermometer. When the mouthpiece is inserted in the mouth, the vital-sign sensor is disposed in a position suited for measurement, as described in detail herein. In some embodiments the probe also includes one or more other vital sign sensors, such as a body temperature sensor, which can be placed in the mouthpiece. Therefore, up to four vital signs - respiratory rate, heart rate, oxygen saturation, and body temperature - can be measured using this novel, easy-to-use probe.

In some exemplary embodiments, a probe includes at least one respiratory sensor in mechanical assembly with a mouthpiece. When the mouthpiece is inserted in the mouth, the respiratory sensors are disposed in a region near the mammalian subject's nose. The mouthpiece can be designed for positional stability of the probe, as described herein. A probe with respiratory sensors fixed to a mouthpiece held stably in the mouth can offer an advantage that the respiratory sensors remain correctly positioned when the mouthpiece is inserted in the mouth, facilitating respiratory rate measurement. The probe can offer an additional advantage that with the mouthpiece in the mouth, the mammalian subject is forced to breathe through the nose and not through the mouth, facilitating respiratory rate measurement. Both advantages are particularly beneficial when the mammalian subject is an infant or young child.

The respiratory sensors of the probe measure physical changes, near the nose, accompanying inhaling and exhaling of the mammalian subject. Some embodiments include two respiratory sensors, one disposed adjacent to each of the mammalian subject's two nostrils. A probe with a respiratory sensor disposed at each nostril can produce more reliable results. For example, if one nostril is partially clogged, the respiratory sensor adjacent to the other nostril will produce a better reading. A respiratory sensor of the probe can be a temperature sensor, such as a thermocouple, thermopile, or thermistor. Alternatively, a respiratory sensor can be a gas absorption sensor or airflow sensor.

In an exemplary embodiment of the invention, the probe includes a pulse oximetry sensor, such as a reflective pulse oximetry sensor, in mechanical connection with a mouthpiece. When the mouthpiece is inserted in the mouth, the pulse oximetry sensor is disposed in a region near the mammalian subject's lip. The pulse oximetry sensor disposed near the lip, in mechanical assembly with the mouthpiece, can offer an advantage that the pulse oximetry sensor remains correctly positioned when the mouthpiece is in the mouth, which can be particularly beneficial when the mammalian subject is an infant or young child.

In some embodiments of the invention, the mouthpiece is designed for positional stability of the probe when placed in the mammalian subject's mouth. For example, the mouthpiece can have a cross-sectional shape that is elongated horizontally along some length. The elongated shape can prevent the mouthpiece from rotating and the attached respiratory sensors and/or pulse oximetry sensor from moving out of position, thereby potentially enhancing measurement accuracy. In some embodiments, the mouthpiece is shaped like a digital oral thermometer, which can offer an additional advantage of measuring the mammalian subject's respiratory rate and/or heart rate and oxygen saturation as easily as taking his temperature with a traditional digital oral thermometer.

In some embodiments, the mouthpiece is shaped like a nipple of a pacifier, potentially improving ease of measurement and comfort to an infant mammalian subject.

In some embodiments of the invention, the probe includes a protrusion. The protrusion is positioned on the probe in front of the mouthpiece (i.e., positioned outside the mouth when the mouthpiece is inserted in the mouth). The protrusion can prevent the portion of the probe in front of the mouthpiece from entering the mouth, advantageously minimizing choking hazard, particularly if the mammalian subject is an infant or young child. The protrusion can be in contact with a lip of the mammalian subject, which can afford additional stability of the probe.

The protrusion can be shaped to conform to the lip or can be flexible, potentially enhancing anatomical conformity and mechanical stability of the probe and facilitating mammalian subject comfort.

In some embodiments, the protrusion houses the respiratory sensors. The protrusion is shaped and sized to dispose the respiratory sensors near the nose.

In some embodiments, the protrusion houses the pulse oximetry sensor. The protrusion includes an opening through which the pulse oximetry sensor can be in contact or close proximity with a lip of the mammalian subject.

In tandem with the mouthpiece, the protrusion can enhance probe stability, thereby facilitating reliable respiratory rate, heart rate, and oxygen saturation measurements.

In some embodiments of the invention, the mouthpiece of the probe further includes a body temperature sensor. The body temperature sensor can be located at a distal tip of the probe (i.e., a tip of the mouthpiece). The body temperature sensor can be inserted into the mouth, possibly under the tongue, in order to measure the mammalian subject's body temperature. A probe with a body temperature sensor can help further to diagnose or monitor the mammalian subject's medical condition.

In an exemplary embodiment of the invention, the method includes measuring a mammalian subject's vital signs. The method is implemented, for example, using a probe that is an embodiment of the invention.

In some embodiments, the method includes analyzing a waveform of physical changes near the mammalian subject's nose to determine frequency of physical changes and calculating the mammalian subject's respiratory rate; for example, by equating the respiratory rate to the frequency.

In some embodiments of the invention, the probe further includes one or more displays, such as LCDs, configured to display some or all measured vital signs.

In some embodiments, the probe is configured to issue an audible tone when one or more indicators have been determined.

In some embodiments, the probe further contains electronic circuitry configured for operation of the probe, such as the following: an analog front-end (AFE) section configured to read the sensors of the probe and provide digital outputs; a processor configured to read the digital outputs, perform the analysis described herein, and control the displays and communication section described herein; and a communication section configured to communicate with one or more external devices, thereby enabling transfer of measurement data to an external device, alerts and reporting of data to the user of an external device, and remote operation of the probe. The communication section may be configured to communicate with nearby devices, remote devices, or both. An example of an external device is a smartphone.

### Detailed Description of embodiments

Non-limiting embodiments of the invention are now described in detail.

### Probe

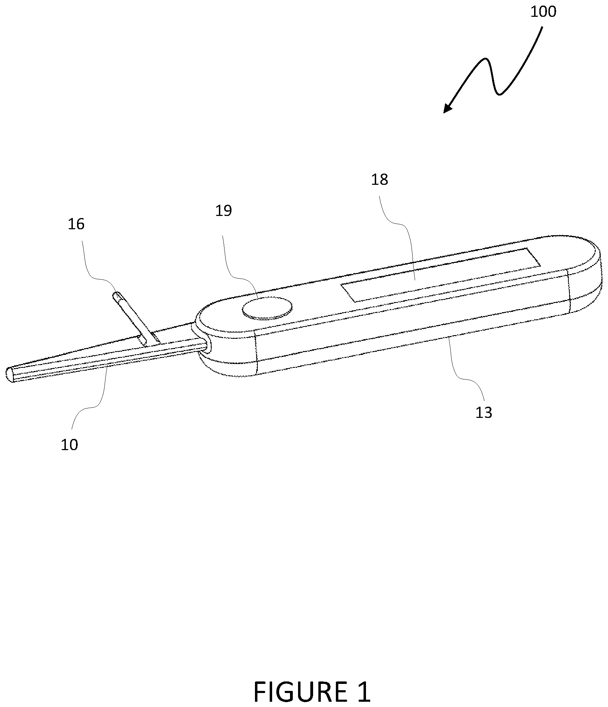

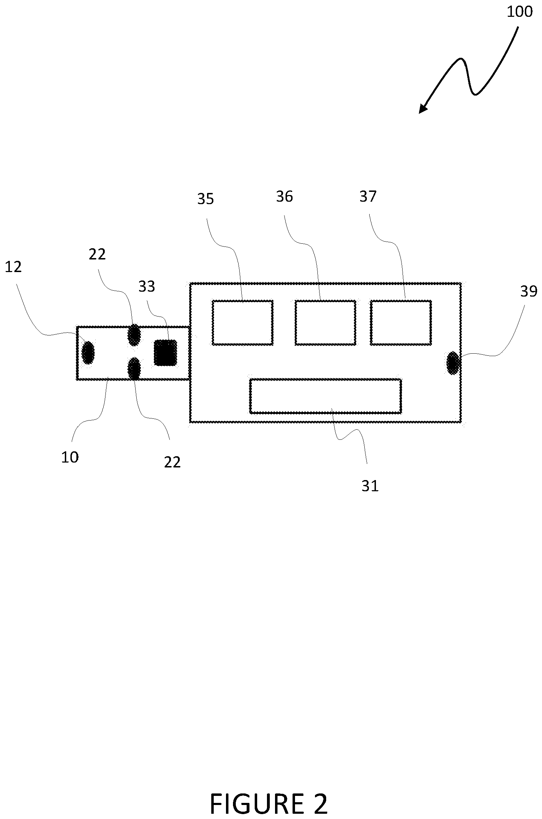

Reference is now made to Figure **1**, an external perspective view of an orally-inserted probe **100** according to some embodiments of the invention. Additionally, reference is made to Figure **2**, a schematic representation of the probe **100**, revealing internal elements of the probe, according to some embodiments of the invention.

In an exemplary embodiment of the invention, a probe **100** for measuring respiratory rate of a mammalian subject includes one or more respiratory sensors **22** - which can be housed in a protrusion **16** of probe **100** - and a mouthpiece **10**. Respiratory sensors **22** are connected to mouthpiece **10**, either directly to mouthpiece **10** or by virtue of mutual assembly in probe **100**, such that respiratory sensors **22** approach the nose when the mouthpiece **10** is inserted into a mouth of the mammalian subject. Probe **100** can include two respiratory sensors, each one positioned near one of the mammalian subject's nostrils. Use of two respiratory sensors positioned near each of the mammalian subject's nostrils can result in a more accurate measurement. For example, during a respiratory rate measurement described herein, if one respiratory sensor **22** gives a better S/N ratio (e.g., one of the mammalian subject's nostrils is partially clogged) can be used.

In some embodiments of the invention, respiratory sensors **22** are temperature sensors. Temperature sensors can be of low mass, allowing a temperature change of temperature sensor to occur within a short time between breaths of the mammalian subject. Temperature sensors can include one or more or thermocouples, thermopiles, or thermistors such as Murata NCP15WB473J03RC.

In some embodiments of the invention, respiratory sensors **22** of probe **100** are connected to an outer casing **13** of probe **100**, arranged such that they will be oriented in a region **28** towards nostrils when mouthpiece **10** is inserted in mouth **26**. Outer casing **13** can contain other features, described herein, of probe **100**. In other embodiments, respiratory sensors are connected directly to mouthpiece **10**. For example, probe 100 can be shaped like a pacifier, making the device smaller and potentially improving ease of measurement and comfort to an infant mammalian subject.

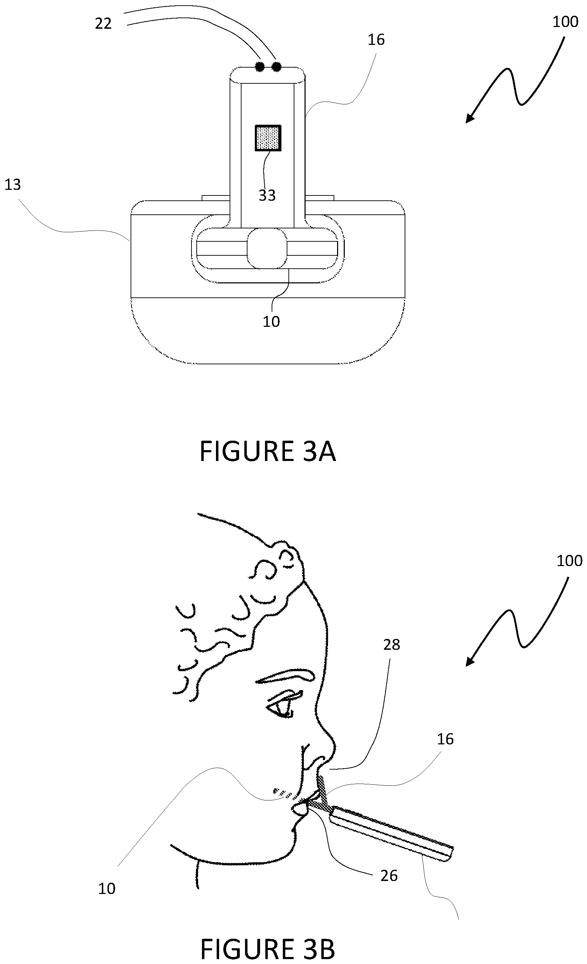

Reference is now also made to Figure **3A**, a front view of probe **100**, and to Figure **3B**, showing probe **100** during measurement, according to some embodiments of the invention.

In some embodiments of the invention, respiratory sensors **22** are housed by a protrusion **16** of probe **100**. Protrusion **16** is at a position on probe **100** and of a shape that disposes the respiratory sensors **22** in a region **28** near the mammalian subject's nostrils when mouthpiece **10** is inserted in the mouth **26**. Protrusion **16** can be in contact with the mammalian subject's lip, which can improve positioning stability of respiratory sensors **22**. Protrusion **16** of probe **100** can be slanted toward the mammalian subject when mouthpiece **10** is inserted in the mouth. Protrusion **16** can be flexible. The slanting and/or flexibility of protrusion **16** can offer anatomical conformity to the mammalian subject's upper lip, potentially providing physical comfort to the mammalian subject while using the probe **100** and also enhancing positional stability of respiratory sensors **22**. Protrusion **16** can provide an obstruction to inserting the portion of probe **100** beyond mouthpiece **10** into the mouth, reducing choking hazard, particularly for infant and child mammalian subjects.

In an exemplary embodiment of the invention, a probe **100** for measuring a mammalian subject's heart rate and oxygen saturation (SpO₂) includes a pulse oximetry sensor **33** and a mouthpiece **10**. In some embodiments, the pulse oximetry sensor **33** is a photo-reflective pulse oximetry sensor optimized for monitoring heart rate and SpO₂, such as the NJL5501R photo-reflector by New Japan Radio Co., Ltd. (Japan).

In some embodiments, pulse oximetry sensor **33** is housed by a protrusion **16** of probe **100**. Protrusion **16** is at a position on probe **100** and of a shape that disposes pulse oximetry sensor **33** facing and proximate the mammalian subject's upper or lower lip when mouthpiece **10** is in the mouth. Conventionally, pulse oximetry measurements are made with a pulse oximetry sensor placed at a mammalian subject's finger or forehead. However, SpO₂ and heart rate measurements were surprisingly found to be equally accurate using a standard reflective pulse oximetry sensor placed near the mammalian subject's lip. In some embodiments, pulse oximetry sensor **33** is housed by a protrusion **16** of the probe **100**. Protrusion **16** of probe **100** can be slanted toward the mammalian subject when mouthpiece **10** is inserted in the mouth. Protrusion **16** can be flexible. The slanting and/or flexibility of protrusion **16** can offer anatomical conformity to the mammalian subject's upper lip, potentially providing physical comfort to the mammalian subject while using the probe **100** and also enhancing positional stability of pulse oximetry sensor **33**. Protrusion **16** can provide an obstruction to inserting the portion of probe **100** beyond mouthpiece **10** into the mouth, reducing choking hazard, particularly for infant and child mammalian subjects.

In some embodiments of the invention, the probe contains both respiratory sensors **22** and a pulse oximetry sensor **33**. Both pulse oximetry sensor and respiratory sensors can be housed in a single protrusion **16** of probe **100**. The protrusion 16 can offer advantages described herein.

In some embodiments of the invention, mouthpiece **10** of probe **100** is shaped to maintain positional stability of the probe **100**. For example, some length of the mouthpiece **10** can be horizontally (i.e., direction of the longest dimension of the lips) elongated in cross-sectional shape. As an additional example, mouthpiece **10** can be shaped like a digital oral thermometer.

In some embodiments of the invention, mouthpiece **10** of probe **100** further contains a body temperature sensor **12**. Body temperature sensor **12** can provide regular measurements of the mammalian subject's body temperature, providing a useful screening tool for diseases associated with increased body temperature, particularly infectious diseases such as pneumonia.

In some embodiments of the invention, probe **100** contains electronic sections. Electronic sections can include an analog front-end section **35**, a processor **36**, a communication section **37**, and a power source **31**. Electronic sections are described in further detail herein.

In some embodiments of the invention, probe **100** includes an external temperature sensor **39**. External temperature sensor **39** can assist in performing internal device calibration and diagnostics of probe **100**.

In some embodiments, probe **100** includes one or more of a display **18**, to display values of measurements including one or more of respiratory rate, body temperature, oxygen saturation, and heart rate. Display **18** can be an LCD-type display. Display **18** can provide those treating the mammalian subject with immediate results without a requirement for communication between probe **100** and an external device. The display can include some form of color coding, such as red for tachypnea, which can be indicative of pneumonia.

In some embodiments, the probe **100** issues an audible signal when a measurement is complete. The audible signal can inform a caregiver that display **18** now shows the measured value of one or more vital signs. The audible signals can differ for different measured vital signs.

In some embodiments, probe **100** includes an on/off switch **19**.

Additional sensors incorporated in the device 100 can include one or more pH sensors and/or enzymatic sensors.

Reference is also now made to Figure **3A**, a front view of probe **100**, from an end including mouthpiece **10**, according to some embodiments of the invention, and Figure **3B**, depicting the probe **100** while in use.

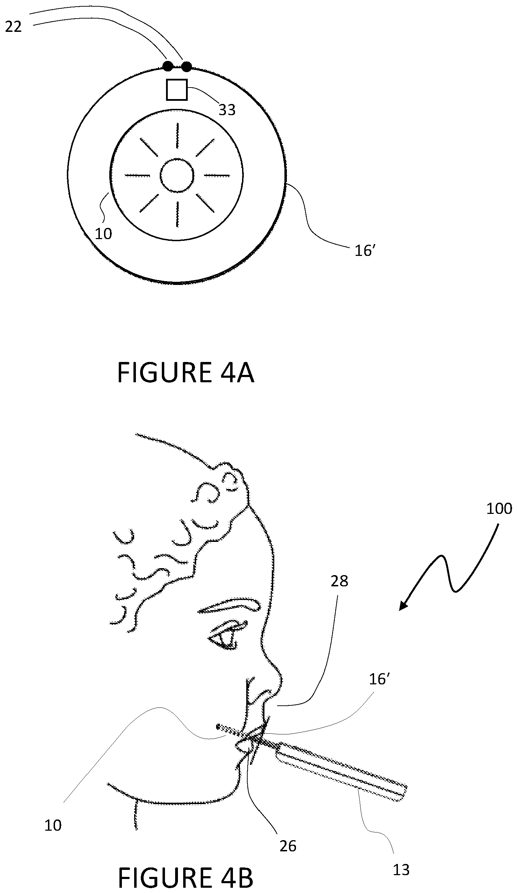

Reference is also now made to Figure **4A**, an end view of probe **100**, from the end including mouthpiece **10**, according to some alternative embodiments of the invention, and Figure **4B**, depicting the probe **100** while in use.

In some embodiments of the invention, probe **100** has a protrusion **16**, described herein, that is slanted toward the mammalian subject when mouthpiece **10** is inserted in the mouth.

In some alternative embodiments of the invention, probe **100** has a skirt **16**', extended around the circumference of mouthpiece **10**, that substantially surrounds the opening of the mouth **26** when mouthpiece **10** is inserted in the mouth of the mammalian subject. Skirt **16**' protrusion offers a relatively large surface area in contact with the mammalian subject around the mammalian subject's lips, which can improve positioning stability of respiratory sensors **22** and/or pulse oximetry sensor of the probe **100**. In some embodiments, skirt **16**' is made of a soft material such as silicone, a gel, or latex rubber, which can enhance anatomical conformity and provide better positioning stability and comfort to the mammalian subject.

A skirt **16**' offers further potential advantages of preventing entrance into the mouth, of the probe beyond the mouthpiece (e.g., minimizing choking hazard) and covering the mouth around the lips so that the mammalian subject is forced to breathe only through his nose, thereby facilitating respiratory sensor measurements.

In some other alternative embodiments, the protrusion or skirt of Figures 3-4 is replaced by a sliding section pushed towards the lip once the mouthpiece is inserted in the mammalian subject's mouth, or incorporated into a flexible arm which presses the probe against the lip.

### Measurement methods

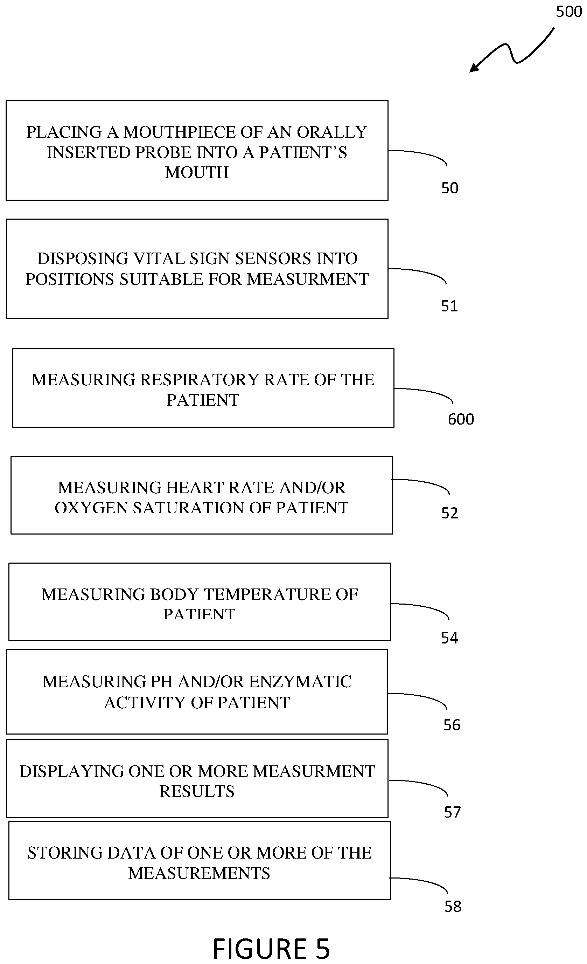

Reference is now made to Figure **5**, showing a method **500** for measuring vitals sign of a mammalian subject according, to some embodiments of the invention.

In an exemplary embodiment, method **500** includes placing a mouthpiece of an orally inserted vital signs probe, such as a probe that is an embodiment of the invention described herein, into a mammalian subject's mouth **50**. Construction of the probe allows that placing the probe effects disposing vital signs sensors in a position suitable for measurement **51**.

For example, disposing vital signs sensors **51** includes placing at least one respiratory sensor near a mammalian subject's nose. The mouthpiece and respiratory sensors are attached such that placing the mouthpiece **50** effects disposing the respiratory sensors near the mammalian subject's nose. Two respiratory sensors can be used, one near each nostril. The respiratory sensors can be temperature sensors. Using two respiratory sensors in proximity to each of the mammalian subject's nostrils can enhance an amplitude of a waveform, described herein, thereby improving the accuracy of the measurement.

Alternatively or additionally, for example, disposing vital signs sensors **51** includes placing a pulse oximetry sensor sensor adjacent to the mammalian subject's upper or lower lip. The mouthpiece and pulse oximetry sensor sensor are attached such that placing the mouthpiece **50** effects disposing the pulse oximetry sensor near the mammalian subject's upper or lower lip.

In an exemplary embodiment, method for measuring vital signs **500** includes measuring respiratory rate of the mammalian subject **600**, described herein.

In an exemplary embodiment, method **500** includes measuring heart rate and/or oxygen saturation of the mammalian subject **52**. For example, using a pulse oximetry sensor that is part of a probe that is an embodiment of the invention, described herein.

In some embodiments, method **500** includes measuring body temperature of the mammalian subject **54**. For example, using a temperature sensor disposed within the mouthpiece of a probe, described herein.

In some embodiments, method **500** includes measuring pH and/or enzymatic activity of a mammalian subject **56**.

In some embodiments, method 500 includes displaying one or more of the measurement results **57**. Displaying **57** can be on one or more displays, described herein, of a probe that is an embodiment of the invention.

In some embodiments, method **500** includes storing data of one or more of the vital signs measurements in memory **59**. The memory can be memory, described herein, of a probe that is an embodiment of the invention. A possible advantage of storing data is better enabling transferring the data to an external device, as described herein. If the data are respiratory sensor readings, storing the data in memory can offer an advantage of enabling a processor of a probe that is an embodiment of the invention to analyze a waveform of respiratory sensor readings and determine respiratory rate, as described herein.

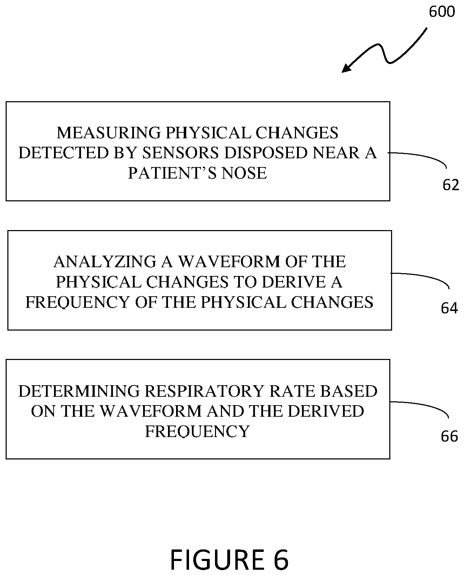

Reference is now made to Figure **6**, showing a method **600** of measuring respiratory rate of a mammalian subject, according to some embodiments of the invention.

In an exemplary embodiment of the invention, method **600** includes measuring physical changes detected by respiratory sensors disposed near a mammalian subject's nose **62**. The physical changes can be caused by the inhaling and exhaling of the mammalian subject. The physical quantity measured can be temperature, or can include, alternatively or in addition, one or more of another quantity, such infrared absorption or air flow. A measurement can be made by comparing the outputs of the respiratory sensors, and selecting the output with strongest signal strength. Selection of the strongest output from several sensors can allow for a more accurate respiratory rate measurement compared with using the output of a single sensor. This is especially advantageous given when comparing the outputs of two sensors, each measuring near a different one of two nostrils of the mammalian subject, as there is always a nostril giving a stronger signal and they tend to alternate every few hours.

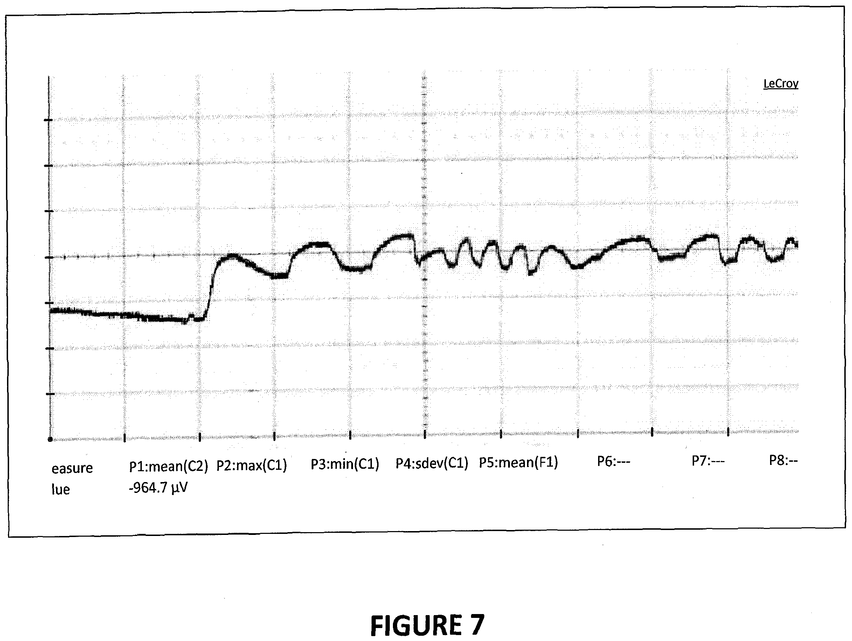

Method **600** further includes analyzing a waveform of the physical changes detected by the sensors, such as the waveform shown in Figure **7**, to derive a frequency of the physical changes **64**. Analyzing **64** can be implemented by a processor of a probe that is an embodiment of the invention, described herein. Alternatively, analyzing **64** can be implemented by an external device, described herein. Analyzing **64** can include using any suitable method, such as computing the Fourier transform of the waveform or an interval of the waveform.

Method **600** further includes determining respiratory rate based on the waveform and the derived frequency **66**. For example, the respiratory rate is set equal to the derived frequency.

### Sharing and utilizing of data

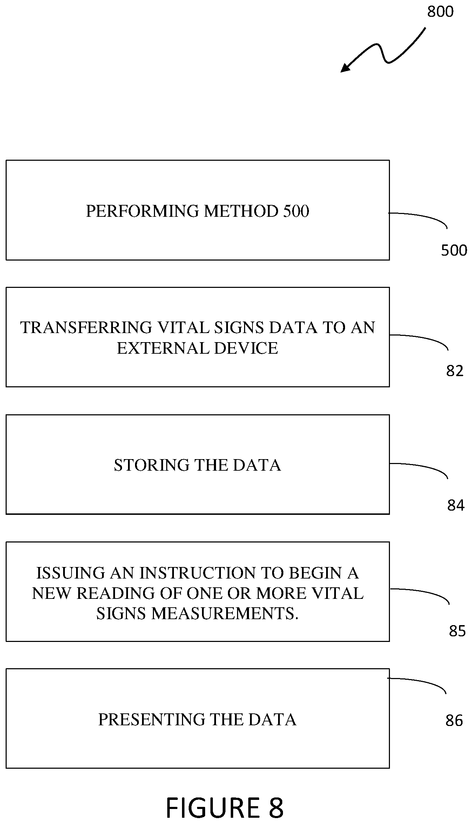

Reference is now made to Figure **8**, showing a method **800** of sharing vital signs data with an external device such as a PC, tablet, wearable device or smartphone and of utilizing the data, according to some embodiments of the invention.

Method **800** includes performing method **500**, measuring vital signs of a mammalian subject, described herein. Method **800** further includes transferring respiratory rate data to an external device **82**. Transferring **82** can be initiated by a processor, descried herein, that is an embodiment of the invention. Transferring **82** may be implemented using a protocol for wireless RF communication. The protocol can be NFC, Wi-Fi, Bluetooth, wireless USB, or other protocols known in the art. Alternatively, or in addition, the protocol can be a cellular technology - which can enable transferring **82** where a nearby device is unavailable - such as GSM, 3G, 4G, or other protocols known in the art. Transmitting data **82** may be implemented, entirely or in part, through a network, such as a LAN, a WAN, the Internet, or a VPN. The external device can be a smartphone, a laptop PC, a router, a web server, or any device with either wired or wireless communication ability. The external device can be located either nearby the mammalian subject or at a remote location. The external device can be in possession, for example, of the mammalian subject, a nearby caregiver, or a remotely located doctor or other medical personnel. Transmitting data can be implemented automatically upon proximity between the probe and external device. Transmitted data may further be retransmitted to a third device; for example, a web server.

The transferred data of step **82** can include respiratory rate measurements. Alternatively, the data can be raw data points of the waveform. Transmitting raw data can enable the step of analyzing the waveform **64** to be performed on the external device or any other device receiving the raw data, alleviating a Processor of the probe, described herein, of this function. Analysis by an external device instead of the probe can, among other advantages, reduce power consumption of the probe. Transmitted data can also include measurements of body temperature, oxygen saturation, and pulse rate. In some embodiments, an application on the external device, or any other device receiving the transmitted data, displays one or more of the measurements, which can allow medical personnel to monitor measurements in real time.

In some embodiments, method **800** further includes storing the data **84**. Storing **84** can be on the external device, or on any other device that may receive the data; for example, a server containing a database of mammalian subject medical records. Storing the data **84** allows, for example, archiving measurements for later viewing a history and historical trends of the mammalian subject's condition, described herein.

In some embodiments, an external device can issue an instruction to begin a new reading of one or more vital signs measurements **84**. The ability of an external device to issue an instruction to begin a reading allows software developers more flexibility in writing applications implementing methods for sharing and utilization of vital-signs measurements, embodiments of methods described herein.

In some embodiments, method **800** further includes presenting the data **86**. Presenting the data **86** can be in the form of alerting a person via the external device or any other device with access to the data. For example, an application on a smartphone of the mammalian subject can alert the mammalian subject of a potentially significant change in the respiratory rate. For further example, a sudden change in body temperature or respiratory rate can cause an alarm to issue on a smartphone or computer of medical personnel, potentially allowing immediate attention and response to the condition.

Presenting the data **86** can entail, for example, an application with access to archived data presenting a history of mammalian subject respiratory rate. A physician can, for example, compare the history and trends at different periods of the mammalian subject's treatment.

### Electronics

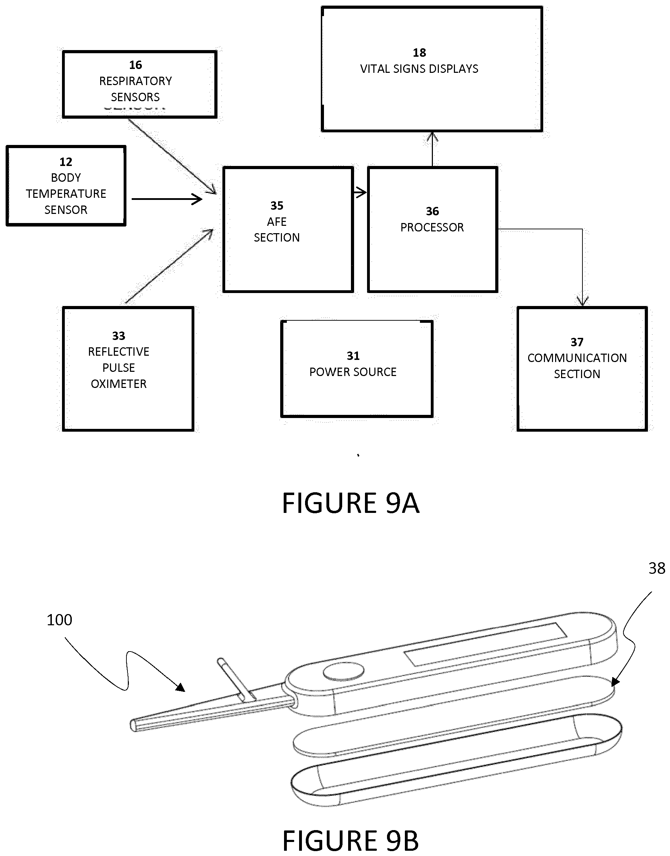

Reference is now made to Figure **9A**, a block diagram of probe **100** according to some embodiments of the invention, revealing the electronic features of the probe and their interoperability with other features of the probe.

In some embodiments, probe **100** includes a power source **31**. Power source **31** can be one or more disposable batteries, such as AA, AAA, coin cell, or button cell batteries. Alternatively, power source can be a rechargeable battery, such as a lithium-ion battery.

In some embodiments, probe **100** includes an AFE section **35** that receives the analog outputs of the vital signs sensors, such as respiratory, pulse oximetry, and body temperature sensors. AFE section **35** contains circuitry for reading and digitizing each sensor output. Circuitry can include, a pre-amplifier, signal conditioning circuit, and A/D converter. The AFE section provides digitized sensor readings, for input into a processor.

In some embodiments, probe **100** contains a processor **36**. Processor **36** receives digital outputs from AFE section **35**. Processor **36** is configured to control functioning of the probe **100**. In some embodiments, processor **36** is configured to perform computations, such as those required for analysis of a respiratory sensor reading waveform, whose method, described herein, is an embodiment of the invention. The processor can be implemented using a CPU, FPGA, or any other information processing components known in the art. In some embodiments, processor **36** contains memory to which the digitized sensor readings and/or computational results can be stored. The memory can be RAM, flash memory, or any other types of memory components known in the art. There can be more than one type of memory, for example serving different purposes such as containing program instructions, short-term data storage, and long-term data storage. Memory is useful, for example, for transmitting data in bulk, e.g., packets, during transfer of respiratory sensor readings from probe **100** to an external device, embodiments described herein.

In some embodiments, probe **100** contains a communication section **37**. Communication section **37** is responsible for implementing communicating data from the probe **100** to an external device. The communicating of data is one feature of a method of sharing and utilizing vital signs data, described herein, that is an embodiment of the invention. The communication section contains the circuitry and components needed for wireless communication, configured to operate according to one or more protocols such as NFC, Wi-Fi, Bluetooth, or wireless USB.

Some functions of communication section **37** can be controlled by processor **36** and other functions of communication section performed controlled internally.

Referring now to Figure **9B**; there is shown a circuit board **38** that can contain one or more of electronic sections: power source **31**, AFE section **35**, Processor **36**, and communication section **37**. The circuit board **38** can be single sided or double sided and can contain multiple layers. The circuit board **38** may in fact comprise several smaller circuit boards, which can facilitate manufacture and test of the electronic sections.

It is understood that the above electronics sections are conceptual only. Some described functions of one or more of the sections may be implemented, in whole or in part, using common components.

## Claims

1. A probe [100] for measuring vital signs of a mammalian subject comprising
a. a mouthpiece [10] configured for insertion into a mouth of said mammalian subject, said mouthpiece comprising a body temperature sensor [12];
b. an extra-oral position-stabilizing part [16, 16'] in mechanical connection with said mouthpiece [10];
c. a reflective pulse oximetry sensor [33] configured to measure heart rate and oxygen saturation of said mammalian subject at such a time as said reflective pulse oximetry sensor [33] is in contact with an outer lip of said mammalian subject;
d. two respiratory sensors [22] configured to measure physical changes near nostrils of said mammalian subject;
e. a processor [36] configured to
i. receive an output of said body temperature sensor [12], outputs of said reflective pulse oximetry sensor [33], and outputs of said two respiratory sensors [22];
ii. analyze a waveform from said outputs of said two respiratory sensors [22];
iii. select one of said waveforms, wherein said selected waveform of one of said two respiratory sensors [22] exhibits a higher signal-to-noise ratio than another of said two respiratory sensors [22]; and
iv. compute a respiratory rate of the subject from said selected waveform; and
f. a display configured to display a body temperature according to said output of said body temperature sensor [12], a heart rate and an oxygen saturation according to said outputs of said reflective pulse oximetry sensor [33], and said respiratory rate;
g. wherein
i. said position-stabilizing part [16, 16'] houses said reflective pulse oximetry sensor [33] and said two respiratory sensors [22], disposing said sensors [33, 22] such that while said mouthpiece [10] is inserted in the mouth,
1. said reflective pulse oximetry sensor [33] is disposed facing said outer lip and in proximity with said outer lip;
2. each of said two respiratory sensors [22] are disposed near each of two nostrils of said mammalian subject;
ii. said position-stabilizing part [16, 16'] is composed of a flexible or soft material and configured to be in anatomical conformity with outer lips of a mammalian subject at such a time as said mouthpiece [10] is inserted in the subject's mouth;
iii. said flexible or soft material and said anatomical conformity with said outer lips of said position-stabilizing part [16, 16'] provide physical comfort to said mammalian subject;
iv. said anatomical conformity of said position-stabilizing part [16, 16'] with said outer lips, in cooperation with said insertion of said mouthpiece [10] in the mouth, stabilizes said dispositions of said photo-reflective pulse oximetry sensor [33] facing and in proximity with said outer lip and of each of said two respiratory sensors [22] near each of two nostrils of the subject;
v. said probe [100] is configured to simultaneously measure said body temperature, heart rate, oxygen saturation, and respiratory rate of said mammalian subject; and
vi. said probe [100] is operable by an untrained caregiver.

2. The probe [100] according to claim 1, wherein said two respiratory sensors [22] comprise two temperature sensors selected from a group consisting of thermocouples, thermopiles, thermistors, and any combination thereof.

3. The probe according to claim 1 or claim 2, wherein said position-stabilizing part is a protrusion [16], wherein said protrusion [16] is configured so that at such time as the mouthpiece [10] is inserted in the mouth, said tip of said protrusion [16] is disposed near the nose, thereby disposing each of said two respiratory sensors [22] in said proximity to each nostril of the mammalian subject.

4. The probe according to claim 1 or claim 2, wherein said position-stabilizing part is a skirt [16'], wherein said skirt [16'] is configured so that at such time as the mouthpiece [10] is inserted in the mouth, said top of the edge of the skirt [16'] is disposed near the nose, thereby disposing each of said two respiratory sensors [22] in said proximity to each nostril of the mammalian subject.

5. The probe according to any of claims 1-4, wherein the processor [36] is further configured to perform said analysis using a Fourier transform of the waveform.

6. The probe according to claim 1, wherein a cross section of at least a portion of said mouthpiece [10] is horizontally elongated, whereby said elongation further stabilizes said dispositions of said respiratory sensors [22] and said reflective pulse oximeter sensors [33].

7. The probe according to claim 1, further comprising a communication section [37] configured to communicate wireless data to an external device, wherein said wireless data comprises one or more results of any of said vital signs measurements and/or said physical changes.

8. The probe according to claim 7, further configured to communicate with an external device via the internet.

9. A method for measuring vital signs of a mammalian subject, comprising
a. providing a probe of claim 1;
b. inserting a mouthpiece of the probe into a mouth of a mammalian subject, said mouthpiece comprising a temperature sensor;
c. disposing an extra-oral position-stabilizing part, made of a flexible or soft material, in anatomical conformity with outer lips of said subject, thereby disposing a reflective pulse oximetry sensor in contact with an outer lip of said mammalian subject and two respiratory sensors near nostrils of said mammalian subject; said pulse-oximetry sensor and said respiratory sensors being housed in said extra-oral position-stabilizing part;
d. receiving, by a processor of said probe, of an output of said temperature sensor, outputs of said reflective pulse oximetry sensor, and outputs of said two respiratory sensors;
e. analyzing, by said processor, a waveform from said outputs of said two respiratory sensors;
f. selecting, by said processor, of one of said waveforms, wherein said selected waveform of one of said two respiratory sensors exhibits a higher signal-to-noise ratio than another of said two respiratory sensors;
g. computing, by said processor, of a respiratory rate of the subject from said selected waveform; and
h. displaying, by a display of said probe, a body temperature according to said output of said temperature sensor, a heart rate and an oxygen saturation according to said outputs of said reflective pulse oximetry sensor, and said respiratory rate;
i. wherein
i. said position-stabilizing part houses said reflective pulse oximetry sensor [33] and said two respiratory sensors [22] such that
1.said reflective pulse oximetry sensor [33] is disposed facing said outer lip and in proximity with said outer lip;
2.each of said two respiratory sensors [22] are disposed near each of two nostrils of said mammalian subject;
ii. said position-stabilizing part is composed of a flexible or soft material and configured to be in anatomical conformity with outer lips of a mammalian subject at such a time as said mouthpiece [10] is inserted in the subject's mouth;
iii. said flexible or soft material and said anatomical conformity with said outer lips of said position-stabilizing part [16, 16'] provide physical comfort to said mammalian subject;
iv. said anatomical conformity of said position-stabilizing part [16, 16'] with said outer lips, in cooperation with said insertion of said mouthpiece [10] in the mouth, stably disposes said photo-reflective pulse oximetry sensor [33] in contact with a said outer lip and each of said two respiratory sensors [22] near each of two nostrils of the subject;
v. said probe [100] is configured to simultaneously measure said body temperature, heart rate, oxygen saturation, and respiratory rate of said mammalian subject; and
vi. said probe [100] is operable by an untrained caregiver.

10. The method according claim 9, wherein said computing of a respiratory rate is made by equating the respiratory rate to said frequency.

11. The method according to claim 9, wherein said measuring physical changes consists of measuring changes in temperature.

12. The method according to claim 9, wherein said analyzing is made using Fourier transforms.

13. The method according to claim 9, further comprising transferring measurement data by a wireless connection of said probe to an external device, said measurement data containing said respiratory rate, said body temperature, said heart rate, said oxygen saturation, said physical changes, or any combination thereof.

14. The probe according to claim 1, wherein said mammalian subject is an infant.

15. The method according to claim 9, wherein said mammalian subject is an infant.

## Patentansprüche

1. Sonde [100] zum Messen von Vitalzeichen eines Säugerindividuums, die aufweist
a. ein Mundstück [10], das zur Einführung in einen Mund des Säugerindividuums konfiguriert ist, wobei das Mundstück einen Körpertemperatursensor [12] aufweist;
b. einen extraoralen Positionsstabilisierungsteil [16, 16'] in mechanischer Verbindung mit dem Mundstück [10];
c. einen Reflexionspulsoximetriesensor [33], der konfiguriert ist, um die Herzfrequenz und die Sauerstoffsättigung des Säugerindividuums in der Zeit zu messen, während der der Reflexionspulsoximetriesensor [33] mit einer äußeren Lippe des Säugerindividuums in Kontakt ist;
d. zwei Atemsensoren [22], die konfiguriert sind, um physische Änderungen in der Nähe von Nasenlöchern des Säugerindividuums zu messen;
e. einen Prozessor [36], der konfiguriert ist, um
i. eine Ausgabe des Körpertemperatursensors [12], Ausgaben des Reflexionspulsoximetriesensors [33] und Ausgaben der beiden Atemsensoren [22] zu empfangen;
ii. eine Wellenform von den Ausgaben der beiden Atemsensoren [22] zu analysieren;
iii. eine der Wellenformen auszuwählen, wobei die ausgewählte Wellenform von einem der beiden Atemsensoren [22] ein höheres Signal-RauschVerhältnis als ein anderer der beiden Atemsensoren [22] aufweist; und
iv. eine Atemfrequenz des Individuums aus der ausgewählten Wellenform zu berechnen; und
f. eine Anzeige, die konfiguriert ist, um eine Körpertemperatur gemäß der Ausgabe des Körpertemperatursensors [12], eine Herzfrequenz und eine Sauerstoffsättigung gemäß den Ausgaben des Reflexionspulsoximetriesensors [33] und die Atemfrequenz anzuzeigen;
g. wobei
i. der Positionsstabilisierungsteil [16, 16'] den Reflexionspulsoximetriesensor [33] und die beiden Atemsensoren [22] aufnimmt, wobei die Sensoren [33, 22] derart angeordnet sind, dass während das Mundstück [10] in den Mund eingeführt ist,
1. der Reflexionspulsoximetriesensor [33] so angeordnet ist, dass er der äußeren Lippe gegenüberliegt und in der Nähe von der äußeren Lippe ist;
2. jeder der beiden Atemsensoren [22] in der Nähe von jedem von zwei Nasenlöchern des Säugerindividuums angeordnet sind;
ii. der Positionsstabilisierungsteil [16, 16'] aus einem flexiblen oder weichen Material besteht und konfiguriert ist, um in anatomischer Übereinstimmung mit äußeren Lippen eines Säugerindividuums in der Zeit zu sein, während der das Mundstück [10] in den Mund des Individuums eingeführt ist;
iii. das flexible oder weiche Material und die anatomische Übereinstimmung mit den äußeren Lippen des Positionsstabilisierungsteiles [16, 16'] physische Behaglichkeit dem Säugerindividuum geben;
iv. die anatomische Übereinstimmung des Positionsstabilisierungsteiles [16, 16'] mit den äußeren Lippen, im Zusammenwirken mit der Einführung des Mundstückes [10] in den Mund die Anordnungen des Lichtreflexionspulsoximetriesensors [33], der der äußeren Lippe gegenüberliegt und in deren Nähe ist, und von jedem der beiden Atemsensoren [22] in der Nähe von zwei Nasenlöchern des Individuums stabilisiert;
v. die Sonde [100] konfiguriert ist, um gleichzeitig die Körpertemperatur, Herzfrequenz, Sauerstoffsättigung und Atemfrequenz des Säugerindividuums zu messen; und
vi. die Sonde [100] von einer ungeschulten Pflegeperson betätigbar ist.

2. Sonde [100] nach Anspruch 1, bei der die beiden Atemsensoren [22] zwei Temperatursensoren aufweisen, die aus einer aus Thermoelementen, Thermopilen, Thermistoren und irgendwelchen Kombinationen daraus bestehenden Gruppe ausgewählt sind.

3. Sonde nach Anspruch 1 oder Anspruch 2, bei der der Positionsstabilisierungsteil ein Vorsprung [16] ist, wobei der Vorsprung [16] so konfiguriert ist, dass in der Zeit, während der das Mundstück [10] in den Mund eingeführt ist, die Spitze des Vorsprungs [16] in der Nähe der Nase angeordnet ist, wodurch jeder der beiden Atemsensoren [22] in der Nähe zu jedem Nasenloch des Säugerindividuums angeordnet wird.

4. Sonde nach Anspruch 1 oder Anspruch 2, bei der der Positionsstabilisierungsteil eine Blende [16'] ist, wobei die Blende [16'] so konfiguriert ist, dass in der Zeit, während der das Mundstück [10] in den Mund eingeführt ist, das obere Ende der Kante der Blende [16'] in der Nähe der Nase angeordnet ist, wodurch jeder der beiden Atemsensoren [22] in der Nähe zu jedem Nasenloch des Säugerindividuums angeordnet wird.

5. Sonde nach einem der Ansprüche 1 - 4, bei der der Prozessor [36] weiter konfiguriert ist, um die Analyse unter Verwendung einer FourierTransformation der Wellenform durchzuführen.

6. Sonde nach Anspruch 1, bei der ein Querschnitt von mindestens einem Teil des Mundstückes [10] horizontal verlängert ist, wodurch die Verlängerung die Anordnungen der Atemsensoren [22] und der Reflexionspulsoximetersensoren [33] weiter stabilisiert.

7. Sonde nach Anspruch 1, ferner mit einem Kommunikationsabschnitt [37], der konfiguriert ist, um drahtlose Daten an ein externes Gerät zu kommunizieren, wobei die drahtlosen Daten ein oder mehrere Ergebnisse von irgendeiner der Vitalzeichenmessungen und/oder der physischen Änderungen aufweisen.

8. Sonde nach Anspruch 7, die ferner konfiguriert ist, um mit einem externen Gerät über das Internet zu kommunizieren.

9. Verfahren zum Messen von Vitalzeichen eines Säugerindividuums, das aufweist
a. Bereitstellen einer Sonde nach Anspruch 1;
b. Einführen eines Mundstückes der Sonde in einen Mund eines Säugerindividuums, wobei das Mundstück einen Temperatursensor aufweist;
c. Anordnen eines extraoralen Positionsstabilisierungsteiles, der aus einem flexiblen oder weichen Material hergestellt ist, in anatomischer Übereinstimmung mit äußeren Lippen des Individuums, wodurch ein Reflexionspulsoximetriesensor in Kontakt mit einer äußeren Lippe des Säugerindividuums und zwei Atemsensoren in der Nähe von Nasenlöchern des Säugerindividuums angeordnet werden; wobei der Pulsoximetriesensor und die Atemsensoren in dem extraoralen Positionsstabilisierungsteil untergebracht sind;
d. durch- einen- Prozessor- der- Sonde- Empfangen einer Ausgabe des Temperatursensors, von Ausgaben des Reflexionspulsoximetriesensors und von Ausgaben der beiden Atemsensoren;
e. durch- den- Prozessor- Analysieren einer Wellenform von den Ausgaben der beiden Atemsensoren;
f. durch- den- Prozessor- Auswählen einer der Wellenformen, wobei die ausgewählte Wellenform von einem der beiden Atemsensoren ein höheres Signal- Rausch- Verhältnis als ein anderer der beiden Atemsensoren aufweist;
g. durch- den- Prozessor- Berechnen einer Atemfrequenz des Individuums aus der ausgewählten Wellenform; und
h. durch- eine- Anzeige- der- Sonde- Anzeigen einer Körpertemperatur gemäß der Ausgabe des Temperatursensors, einer Herzfrequenz und einer Sauerstoffsättigung gemäß den Ausgaben des Reflexionspulsoximetriesensors, und der Atemfrequenz;
i. wobei
i. der Positionsstabilisierungsteil den Reflexionspulsoximetriesensor [33] und die beiden Atemsensoren [22] derart aufnimmt, dass
1. der Reflexionspulsoximetriesensor [33] gegenüber der äußeren Lippe und in der Nähe der äußeren Lippe angeordnet ist;
2. jeder der beiden Atemsensoren [22] in der Nähe von jedem von zwei Nasenlöchern des Säugerindividuums angeordnet sind;
ii. der Positionsstabilisierungsteil aus einem flexiblen oder weichen Material besteht und konfiguriert ist, um in anatomischer Übereinstimmung mit äußeren Lippen eines Säugerindividuums in einer Zeit zu sein, während der das Mundstück [10] in den Mund des Individuums eingeführt ist;
iii. das flexible oder weiche Material und die anatomische Übereinstimmung mit den äußeren Lippen des Positionsstabilisierungsteiles [16, 16'] physische Behaglichkeit dem Säugerindividuum geben;
iv. die anatomische Übereinstimmung des Positionsstabilisierungsteiles [16, 16'] mit den äußeren Lippen im Zusammenwirken mit der Einführung des Mundstückes [10] in den Mund den Lichtreflexionspulsoximetriesensor [33] in Kontakt mit einer äußeren Lippe und jeden der beiden Atemsensoren [22] in der Nähe von jedem von zwei Nasenlöchern des Individuums stabil anordnet;
v. die Sonde [100] konfiguriert ist, um gleichzeitig die Körpertemperatur, Herzfrequenz, Sauerstoffsättigung und Atemfrequenz des Säugerindividuums zu messen; und
vi. die Probe [100] von einer ungeschulten Pflegeperson betätigbar ist.

10. Verfahren nach Anspruch 9, bei dem das Berechnen einer Atemfrequenz durch Gleichsetzen der Atemfrequenz mit der Frequenz durchgeführt wird.

11. Verfahren nach Anspruch 9, bei dem das Messen von physischen Änderungen im Messen von Temperaturänderungen besteht.

12. Verfahren nach Anspruch 9, bei dem das Analysieren unter Verwendung von Fourier-Transformationen durchgeführt wird.

13. Verfahren nach Anspruch 9, das ferner das Übertragen von Messdaten durch eine drahtlose Verbindung der Sonde zu einem externen Gerät aufweist, wobei die Messdaten die Atemfrequenz, die Körpertemperatur, die Herzfrequenz, die Sauerstoffsättigung, die physischen Änderungen oder irgendwelche Kombinationen daraus enthalten.

14. Sonde nach Anspruch 1, bei der das Säugerindividuum ein Kind ist.

15. Verfahren nach Anspruch 9, bei dem das Säugerindividuum ein Kind ist.

## Revendications

1. Sonde [100] pour mesurer les signes vitaux d'un sujet mammifère comprenant
a. un embout buccal [10] configuré pour être inséré dans la bouche dudit sujet mammifère, ledit embout buccal comprenant un capteur de température corporelle [12] ;
b. une partie de stabilisation de position extra-buccale [16, 16'] en raccordement mécanique avec ledit embout buccal [10] ;
c. un capteur d'oxymétrie de pouls par réflexion [33] configuré pour mesurer la fréquence cardiaque et la saturation en oxygène dudit sujet mammifère à un moment où ledit capteur d'oxymétrie de pouls par réflexion [33] est en contact avec une lèvre extérieure dudit sujet mammifère ;
d. deux capteurs respiratoires [22] configurés pour mesurer les changements physiques à proximité des narines dudit sujet mammifère ;
e. un processeur [36] configuré pour
i. recevoir une sortie dudit capteur de température corporelle [12], des sorties dudit capteur d'oxymétrie de pouls par réflexion [33], et des sorties desdits deux capteurs respiratoires [22] ;
ii. analyser une forme d'onde desdites sorties desdits deux capteurs respiratoires [22] ;
iii. sélectionner l'une desdites formes d'onde, dans laquelle ladite forme d'onde sélectionnée de l'un desdits deux capteurs respiratoires [22] présente un rapport signal/bruit supérieur à un autre desdits deux capteurs respiratoires [22] ; et
iv. calculer une fréquence respiratoire du sujet à partir de ladite forme d'onde sélectionnée ; et
f. un affichage configuré pour afficher une température corporelle conformément à ladite sortie dudit capteur de température corporelle [12], une fréquence cardiaque et une saturation en oxygène conformément auxdites sorties dudit capteur d'oxymétrie de pouls par réflexion [33], et ladite fréquence respiratoire ;
g. dans laquelle
i. ladite partie de stabilisation de position [16, 16'] reçoit ledit capteur d'oxymétrie de pouls par réflexion [33] et lesdits deux capteurs respiratoires [22], disposant lesdits capteurs [33, 22] de telle sorte qu'alors que ledit embout buccal [10] est inséré dans la bouche,
1. ledit capteur d'oxymétrie de pouls par réflexion [33] est disposé face à ladite lèvre extérieure et à proximité de ladite lèvre extérieure ;
2. chacun desdits deux capteurs respiratoires [22] est disposé à proximité de chacune des deux narines dudit sujet mammifère ;
ii. ladite partie de stabilisation de position [16, 16'] est composée d'un matériau flexible ou souple et configurée pour être en conformité anatomique avec les lèvres extérieures d'un sujet mammifère au moment où ledit embout buccal [10] est inséré dans la bouche du sujet ;
iii. ledit matériau flexible ou souple et ladite conformité anatomique avec lesdites lèvres extérieures de ladite partie de stabilisation de position [16, 16'] fournissent un confort physique audit sujet mammifère;
iv. ladite conformité anatomique de ladite partie de stabilisation de position [16, 16'] avec lesdites lèvres extérieures, en coopération avec ladite insertion dudit embout buccal [10] dans la bouche, stabilise lesdites dispositions dudit capteur d'oxymétrie de pouls par photo-réflexion [33] tourné vers et à proximité de ladite lèvre extérieure et de chacun desdits deux capteurs respiratoires [22] à proximité de chacune des deux narines du sujet ;
v. ladite sonde [100] est configurée pour simultanément mesurer lesdites température corporelle, fréquence cardiaque, saturation en oxygène, et fréquence respiratoire dudit sujet mammifère ; et
vi. ladite sonde [100] peut être utilisée par un personnel soignant non qualifié.

2. Sonde [100] selon la revendication 1, dans laquelle lesdits deux capteurs respiratoires [22] comprennent deux capteurs de température sélectionnés dans un groupe constitué par des thermocouples, des thermopiles, des thermistors, et des combinaisons de ceux-ci.

3. Sonde selon la revendication 1 ou la revendication 2, dans laquelle ladite partie de stabilisation de position est une saillie [16], dans laquelle ladite saillie [16] est configurée de manière à ce qu'au moment où l'embout buccal [10] est inséré dans la bouche, ladite pointe de ladite saillie [16] est disposée à proximité du nez, disposant ainsi chacun desdits deux capteurs respiratoires [22] dans ladite proximité de chaque narine du sujet mammifère.

4. Sonde selon la revendication 1 ou la revendication 2, dans laquelle ladite partie de stabilisation de position est une jupe [16'], dans laquelle ladite jupe [16'] est configurée de telle sorte qu'au moment où l'embout buccal [10] est inséré dans la bouche, ladite partie supérieure du bord de la jupe [16'] est disposée à proximité du nez, disposant ainsi chacun desdits deux capteurs respiratoires [22] dans ladite proximité de chaque narine du sujet mammifère.

5. Sonde selon l'une quelconque des revendications 1 à 4, dans laquelle le processeur [36] est en outre configuré pour réaliser ladite analyse en utilisant une transformée de Fourier de la forme d'onde.

6. Sonde selon la revendication 1, dans laquelle une section transversale d'au moins une partie dudit embout buccal [10] est horizontalement allongée, moyennant quoi ledit allongement stabilise davantage lesdites dispositions desdits capteurs respiratoires [22] et dudit capteur d'oxymétrie de pouls par réflexion [33].

7. Sonde selon la revendication 1, comprenant en outre une section de communication [37] configurée pour communiquer des données sans fil à un dispositif externe, dans laquelle lesdites données sans fil comprennent un ou plusieurs résultats de l'un quelconque desdites mesures des signes vitaux et/ou desdits changements physiques.

8. Sonde selon la revendication 7, en outre configurée pour communiquer avec un dispositif externe par l'intermédiaire d'Internet.

9. Procédé de mesure de signes vitaux d'un sujet mammifère, comprenant
a. la fourniture d'une sonde selon la revendication 1 ;
b. l'insertion d'un embout buccal de la sonde dans la bouche d'un sujet mammifère, ledit embout buccal comprenant un capteur de température ;
c. la disposition d'une partie de stabilisation de position extra-buccale, formée en un matériau flexible ou souple, en conformité anatomique avec lesdites lèvres extérieures dudit sujet, disposant ainsi un capteur d'oxymétrie de pouls par réflexion en contact avec une lèvre extérieure dudit sujet mammifère et deux capteurs respiratoires à proximité des narines dudit sujet mammifère ; ledit capteur d'oxymétrie de pouls et lesdits capteurs respiratoires étant reçus dans ladite partie de stabilisation extra-buccale ;
d. la réception, par un processeur de ladite sonde, d'une sortie dudit capteur de température, des sorties dudit capteur d'oxymétrie de pouls par réflexion, et des sorties desdits deux capteurs respiratoires ;
e. l'analyse, par ledit processeur, d'une forme d'onde desdites sorties desdits deux capteurs respiratoires ;
f. la sélection, par ledit processeur, de l'une desdites formes d'onde, dans lequel ladite forme d'onde sélectionnée de l'un desdits deux capteurs respiratoires présente un rapport signal/bruit supérieur à un autre desdits deux capteurs respiratoires ;
g. le calcul, par ledit processeur, d'une fréquence respiratoire du sujet à partir de ladite forme d'onde sélectionnée ; et
h. l'affichage, par un affichage de ladite sonde, d'une température corporelle conformément à ladite sortie dudit capteur de température, d'une fréquence cardiaque et d'une saturation en oxygène conformément auxdites sorties dudit capteur d'oxymétrie de pouls par réflexion, et de ladite fréquence respiratoire ;
i. dans lequel
i. ladite partie de stabilisation de position reçoit ledit capteur d'oxymétrie de pouls par réflexion [33] et lesdits deux capteurs respiratoires [22] de telle sorte que
1. ledit capteur d'oxymétrie de pouls par réflexion [33] est disposé face à ladite lèvre extérieure et à proximité de ladite lèvre extérieure ;
2. chacun desdits deux capteurs respiratoires [22] est disposé à proximité de chacune des deux narines dudit sujet mammifère ;
ii. ladite partie de stabilisation de position est composée d'un matériau flexible ou souple et configurée pour être en conformité anatomique avec les lèvres extérieures d'un sujet mammifère au moment où ledit embout buccal [10] est inséré dans la bouche du sujet ;
iii. ledit matériau flexible ou souple et ladite conformité anatomique avec lesdites lèvres extérieures de ladite partie de stabilisation de position [16, 16'] fournissent un confort physique audit sujet mammifère ;
iv. ladite conformité anatomique de ladite partie de stabilisation de position [16, 16'] avec lesdites lèvres extérieures, en coopération avec ladite insertion dudit embout buccal [10] dans la bouche, dispose stablement ledit capteur d'oxymétrie de pouls par photo-réflexion [33] en contact avec ladite lèvre extérieure et chacun desdits deux capteurs respiratoires [22] à proximité de chacune des deux narines du sujet ;
v. ladite sonde [100] est configurée pour simultanément mesurer lesdites température corporelle, fréquence cardiaque, saturation en oxygène, et fréquence respiratoire dudit sujet mammifère ; et
vi. ladite sonde [100] peut être utilisée par un personnel soignant non qualifié.

10. Procédé selon la revendication 9, dans lequel ledit calcul d'une fréquence respiratoire est réalisé en établissant une équivalence entre la fréquence respiratoire et ladite fréquence.

11. Procédé selon la revendication 9, dans lequel ladite mesure des changements physiques consiste à mesurer les changements de température.

12. Procédé selon la revendication 9, dans lequel ladite analyse est réalisée en utilisant des transformées de Fournier.

13. Procédé selon la revendication 9, comprenant en outre le transfert de données de mesures par une connexion sans fil de ladite sonde à un dispositif externe, lesdites données de mesures contenant ladite fréquence respiratoire, ladite température corporelle, ladite fréquence cardiaque, ladite saturation en oxygène, lesdits changements physiques ou toute combinaison de ceux-ci.

14. Sonde selon la revendication 1, dans laquelle ledit sujet mammifère est un nourrisson.

15. Procédé selon la revendication 9, dans lequel ledit sujet mammifère est un nourrisson.
